# EUROPEAN PATENT SPECIFICATION

(11) **EP 4 328 258 B1**
(45) Date of publication and mention of the grant of the patent: **18.11.2026**
(21) Application number: 22791015.5
(22) Date of filing: 19.04.2022
(51) Int. Cl.: C08J 3/075, C08J 3/24, A61L 27/20, A61L 27/50, A61L 27/52

(54) **THIOLATED POLYSACCHARIDE DERIVATIVE HYDROGEL, PREPARATION METHOD THEREFOR, AND APPLICATION THEREOF**
THIOLIERTES POLYSACCHARIDDERIVATHYDROGEL, HERSTELLUNGSVERFAHREN DAFÜR UND ANWENDUNG DAVON
HYDROGEL DÉRIVÉ DE POLYSACCHARIDE THIOLÉ, SON PROCÉDÉ DE PRÉPARATION ET SON APPLICATION

(30) Priority: 20.04.2021 CN 202110433689; 20.04.2021 CN 202110433699
(43) Date of publication of application: 28.02.2024
(73) Proprietor: QINGDAO HEALTH OCEAN BIOPHARMACEUTICAL CO., LTD., Laoshan District Qingdao, Shandong 266104 (CN)
(72) Inventor: HAN, Baoqin, Qingdao, Shandong 266003 (CN); WANG, Shuo, Qingdao, Shandong 266003 (CN); LIU, Wanshun, Qingdao, Shandong 266003 (CN); YU, Shuqin, Qingdao, Shandong 266003 (CN)
(74) Representative: Vossius & Partner Patentanwälte Rechtsanwälte mbB
(86) International application number: PCT/CN2022/087577
(87) International publication number: WO 2022/222907

(56) References cited:
- EP-A2- 2 614 828
- CN-A- 101 367 884
- CN-A- 102 399 295
- CN-A- 102 399 295
- CN-A- 108 324 680
- CN-A- 112 842 929
- CN-A- 113 174 063
- CN-A- 113 185 716
- CN-A- 113 214 505
- YU SHUQIN ET AL: "Injectable self-crosslinking hydrogels based on hyaluronic acid as vitreous substitutes", INTERNATIONAL JOURNAL OF BIOLOGICAL MACROMOLECULES, ELSEVIER BV, NL, vol. 208, 14 March 2022 (2022-03-14), pages 159 - 171, XP087044454, ISSN: 0141-8130, [retrieved on 20220314], DOI: 10.1016/J.IJBIOMAC.2022.03.046
- SCHNICHELS SVEN ET AL: "Efficacy of two different thiol-modified crosslinked hyaluronate formulations as vitreous replacement compared to silicone oil in a model of retinal detachment", PLOS ONE, vol. 12, no. 3, 1 March 2017 (2017-03-01), pages e0172895, XP055882359, DOI: 10.1371/journal.pone.0172895
- SANTHANAM SRUTHI ET AL: "Investigating thiol-modification on hyaluronan via carbodiimide chemistry using response surface methodology", vol. 103, no. 7, 18 November 2014 (2014-11-18), US, pages 2300 - 2308, XP093181222, ISSN: 1549-3296, Retrieved from the Internet <URL:https://www.ncbi.nlm.nih.gov/pmc/articles/PMC5527969/pdf/nihms640828.pdf> DOI: 10.1002/jbm.a.35366
- LI YI, SHE YING-QI， ZHU HE-KANG， ZHAO YU-YING， XIE BIAO-MING， PENG ZHI-PING : " Synthesis of Ｒedox-responsive Thiolated Chitosan Hydrogel with Disulfide Cross-links for Controlled Ｒelease of a Model Protein ", FINE CHEMICALS, vol. 34, no. 1, 15 January 2017 (2017-01-15), pages 28 - 33, XP055980249, ISSN: 1003-5214

## Description

### FIELD OF THE INVENTION

The present invention relates to a biomedical material for ophthalmology, particularly to a thiolated hyaluronic acid polysaccharide derivative hydrogel and a thiolated chitin/chitosan derivative hydrogel, as well as to the preparation methods therefor and applications thereof.

### BACKGROUND OF THE INVENTION

The vitreous body is a colorless, transparent gel-like substance that fills the space between the lens and the retina within the eye. It occupies more than 2/3 of the volume of the eyeball and is mainly composed of water, collagen protein, hyaluronic acid, and proteoglycans. Water constitutes about 99% of the volume of the vitreous body. Hyaluronic acid and collagen protein interact randomly and bind with water to maintain the stability of the vitreous body. The vitreous body has high transparency, allowing more than 90% of visible light to pass through. It provides support to the retina and protects the surrounding eye tissues, allowing light to reach the sensory receptors at the back of the eye. It plays an important role in maintaining the shape of the eyeball, regulating intraocular oxygen tension, providing shock absorption, and maintaining the position of the retina and lens. Vitrectomy is an important ophthalmic surgery used to treat eye disorders such as retinal diseases and retinal detachment. After vitrectomy, the vitreous cavity needs to be filled with a vitreous substitute to maintain the shape of the eye and provide support to the retina. Therefore, the vitreous substitute is an important factor affecting the effectiveness of the surgery.

Currently, commonly used vitreous substitutes include gas, silicone oil, and perfluorocarbon liquid. These substitutes are used for vitreous filling in different surgical situations but still have various complications. For example, gas filling has a short duration and may cause lens opacity. Perfluorocarbon liquid is highly toxic, while silicone oil is prone to emulsification and has some toxicity to eye tissues, often causing complications such as glaucoma and cataracts. Elevated intraocular pressure after vitreous filling is a common occurrence in clinical practice. Severe high intraocular pressure can cause ischemia and vision loss, while long-term and chronic high intraocular pressure can also damage the optic nerve.

As a substitute for the vitreous body, high translucency, non-toxicity to eye tissues, and good viscoelasticity are extremely important. Patent application CN1964723A discloses small particles with an average size of 0.01-500µm prepared by cross-linking large molecule compounds with a cross-linking agent. A liquid formulation containing these tiny particles is injected into the eye to improve the visibility of transparent eye tissues by the light scattering properties of the surface of these particles, making surgical visual field detection easier for surgeries involving vitreous body removal. However, these small particles with light scattering properties have poor transparency, so they cannot be used as vitreous substitutes.

Researchers have attempted to develop artificial vitreous substitutes using hydrophilic polymer hydrogels such as polyethylene glycol, polyvinyl alcohol, or polyvinylpyrrolidone. However, there are still several issues, such as poor biological compatibility within the eye and short degradation and absorption time. In terms of non-toxicity to eye tissues, natural biomacromolecules usually have advantages over synthetic polymer compounds, as they have better biocompatibility with biological tissues and can be degraded and absorbed in vivo without producing toxic byproducts. Chinese patent application CN102762647A discloses a hydrogel formed by cross-linking oxidized hyaluronic acid and dioxazoline, which can be used as a substitute for the eye's vitreous body. However, this patent application also has limitations. On one hand, the oxidation of hyaluronic acid inevitably reduces its molecular weight, and on the other hand, the Schiff base formed by cross-linking oxidized hyaluronic acid and dioxazoline is prone to hydrolysis, resulting in poor stability.

Patent application CN104761735A discloses the cross-linking of hyaluronic acid with polyglutamic acid and heparin using a cross-linking agent to enhance the enzymatic resistance of the hyaluronic acid.

With respect to bond stability, disulfide bond is better than Schiff base. In patent application CN104892962A, thiolated hyaluronic acid was prepared. By adjusting density of the thiol group, gelation temperature, molecular weight and other factors, the gelation time and mechanical properties of the hydrogel were controlled. The obtained thiolated hyaluronic acid hydrogel had a storage modulus in the range of 3.3kPa to 42.8kPa with a strain of 1%. However, this hydrogel is too rigid and hard to be suitable as a substitute for the vitreous body. It has been reported that as the thiol substitution degree increases, the self-crosslinking ability of the thiolated hyaluronic acid hydrogel improves, but at the same time, the rigidity of the hydrogel also increases, leading to poor flexibility. However, if the thiol substitution degree is too low, the self-crosslinking ability of the hyaluronic acid is poor, resulting in fragile gels and poor resilience thereof.

The vitreous body of the eye is a highly transparent, flexible gel-like elastic material. As a substitute for the vitreous body, its main functions are to maintain the shape of the eyeball, provide shock absorption, and keep the retina in its correct position. Therefore, the substitute material needs to have properties such as flexibility, compressive elasticity, and good transparency. At the same time, after vitreous surgery, when it is filled into the vitreous cavity, it should exert sufficient pressure on the retina to prevent retinal detachment. However, there is currently no research reported on what properties of hydrogels are suitable as substitutes for the vitreous body. From an application perspective, if the hydrogel is too rigid and has minimal compressive deformation, its cushioning and shock absorption will be poor, which may harm the eye tissues and alter the axial length of the eye. If the hydrogel degrades too quickly in the body, the pressure exerted on the retina will be short-lived, making retinal detachment more likely to occur.

SCHNICHELS SVEN ET AL: "Efficacy of two different thiol-modified crosslinked hyaluronate formulations as vitreous replacement compared to silicone oil in a model of retinal detachment", PLOS ONE,vol. 12, no. 3, 1 March 2017, page e0172895, XP055882359 relates to thiol modified crosslinked hyaluronate based hydrogel as vitreous replacement.

### SUMMARY OF THE INVENTION

The scope of protection is defined by the claims. Embodiments in the description relating to methods of treatment are not covered by the claims. Any "embodiment" or " example"which is disclosed in the description but is not covered by the claims should be considered as presented for illustrative purpose only.

In view of the above problems, the first object of the present invention is to provide a thiolated polysaccharide derivative hydrogel, particularly a thiolated hyaluronic acid derivative hydrogel or a thiolated chitin/chitosan derivative hydrogel, which has high transparency, good flexibility, easy compression resilience, non-toxicity to ocular tissues, and resistance to degradation, and can be used as a substitute for vitreous body to overcome the shortcomings of the existing technology.

Another object of the present invention is to provide a method for preparing a thiolated polysaccharide derivative hydrogel, especially a method for preparing a thiolated hyaluronic acid polysaccharide derivative hydrogel or a thiolated chitin/chitosan derivative hydrogel, as well as the use of the thiolated hyaluronic acid polysaccharide derivative hydrogel or the thiolated chitin/chitosan derivative hydrogel in the manufacture of vitreous substitute.

The reported use of thiolated hyaluronic acid hydrogels mainly focuses on the gelation properties thereof. Among them, the higher the thiol substitution degree, the better the self-crosslinking gelation properties, but at the same time, the rigidity of the hydrogel increases, leading to poor flexibility. However, if the thiol substitution degree of thiolated hyaluronic acid is too low, the self-crosslinking gelation properties are poor, resulting in a fragile gel and poor resilience.

The hyaluronic acid derivatives selected in the present invention are derivatives with side chains grafted onto the hydroxyl and carboxyl groups of the hyaluronic acid molecules. Compared with hyaluronic acid without grafted side chains, the presence of side chain groups in the hyaluronic acid derivatives increases the mutual support between hyaluronic acid molecules, both intermolecularly and intramolecularly. By moderately thiolating the hyaluronic acid derivatives, a thiolated hyaluronic acid polysaccharide derivative hydrogel is prepared. Due to the presence of side chain groups in the hydrogel and an appropriate degree of thiol substitution, the interactions between molecules, both intermolecularly and intramolecularly, are increased in the hydrogel, which can enhance the flexibility of the hydrogel while maintaining good resilience and gel form, achieving the purpose of being suitable as a vitreous substitute.

The chitin/chitosan derivative selected in the present invention is a water-soluble derivative obtained by grafting side chain groups onto the hydroxyl and amino positions of chitin/chitosan molecules. The resulting hydrogel, due to the presence of side chain groups, has increased mutual support between chitin/chitosan derivatives in the hydrogel, thereby enhancing the flexibility and compression resilience of the hydrogel. By using the chitin derivative and the chitosan derivative with grafted side chain groups as raw materials for the thiolation reaction, hydrogels with better degradation resistance can be obtained due to the better stability of the disulfide bond compared with cysteine. In addition, chitosan and chitosan are from D-N-acetylglucosamine and D-glucosamine, respectively, by β-1,4 glycoside bonds. Beta-1,4 glycoside bonds have better degradation resistance than beta-1,3 glycoside bonds, so the degradation resistance of chitin/chitosan is even better than that of hyaluronic acid. Moreover, the water-soluble derivatives of chitin/chitosan have good biocompatibility.

The technical solution of the present invention simultaneously considers the balance between the flexibility, resilience, and degradation resistance of the hydrogel used as a vitreous substitute. By using hyaluronic acid derivative with grafted side chain groups as raw materials for thiolating reaction and controlling an appropriate thiol substitution degree, the interactions between molecules, both intermolecularly and intramolecularly, in the thiolated hyaluronic acid polysaccharide derivative hydrogel are increased, thereby enhancing the flexibility and compression resilience of the hydrogel and maintaining the gel form properties. By using the chitin derivatives and the chitosan derivatives with grafted side chain groups as raw materials for thiolating reaction and controlling an appropriate thiol substitution degree, the interactions between molecules, both intermolecularly and intramolecularly, in the thiolated chitin/chitosan derivative hydrogel are increased, thereby enhancing the degradation resistance, flexibility, and compression resilience of the hydrogel and maintaining the gel form characteristics, achieving the purpose of being suitable as a vitreous substitute.

To achieve the above purpose, the present invention relates to a thiolated polysaccharide derivative hydrogel for use as an intraocular vitreous substitute according to claim 1. The hydrogel is selected from: (i) a hydrogel formed from a thiolated hyaluronic acid derivative and an aqueous solvent, wherein the mass percentage concentration of the thiolated hyaluronic acid derivative in the aqueous solvent is 3% to 6%, and the thiol substitution degree of the thiolated hyaluronic acid derivative is 3% to 20%, and wherein the hyaluronic acid derivative is selected from the group consisting of hydroxyethyl hyaluronic acid, hydroxypropyl hyaluronic acid, acetyl hyaluronic acid, butyryl hyaluronic acid, aminoacetic hyaluronic acid, aminopropionic hyaluronic acid, aminobutyric hyaluronic acid, and hydrazide hyaluronic acid; and
(ii) a hydrogel formed from a thiolated chitin/chitosan derivative and an aqueous solvent, wherein the mass percentage concentration of the thiolated chitin/chitosan derivative in the aqueous solvent is 2% to 10%, and the thiol substitution degree of the thiolated chitin/chitosan derivative is 3% to 25%, and wherein the chitin/chitosan derivative is selected from the group consisting of carboxyethyl chitosan, hydroxyethyl chitosan, hydroxypropyl chitosan, hydroxybutyl chitosan, succinyl chitosan, aminoacetic chitosan, aminopropionic chitosan, aminobutyric chitosan, hydroxyethyl succinyl chitosan, hydroxypropyl succinyl chitosan, hydroxybutyl succinyl chitosan, carboxymethyl chitin, and carboxyethyl chitin.

The aqueous solvent is selected from the group consisting of water, physiological saline, phosphate buffer, physiological balanced solution and glucose solution.

The method for preparing the hydrogel as described above ( not according to the claimed invention) includes the following steps:
(1) dissolving a polysaccharide derivative in water or DMSO solvent with stirring;
(2) adding carboxy-activating agents 1-(3-dimethylaminopropyl)-3-ethylcarbodiimide hydrochloride (EDC) and N-hydroxy succinimide (NHS) to the polysaccharide derivative solution to form a reaction system, adjusting the pH of the reaction system to 4-6, and stirring it for 0.5 to 3 h;
(3) adding a thiolating agent to the reaction system of step (2), adjusting the pH of the reaction system to 3.5 - 6, stirring it under dark condition to obtain a reaction product;
(4) performing dialysis of the reaction product in acidic aqueous solution under dark condition and freeze-drying the resulting solution; or precipitating the reaction product with ethanol, washing, precipitating with ethanol, dehydrating, and drying under vacuum; to obtain a thiolated polysaccharide derivative, which is stored with sealing under dark condition at low temperature; and
(5) dissolving the thiolated polysaccharide derivative in an aqueous solvent to prepare a gel solution with a concentration of 2-10%, adjusting the pH to 7.0-8.5, wherein the gel solution is subjected to self-crosslinking to obtain a hydrogel.

According to the method of claim 5, wherein the polysaccharide derivative is a hyaluronic acid derivative, and in step (5), the thiolated hyaluronic acid derivative is dissolved in an aqueous solvent to prepare a gel solution with a concentration of 3-6% adjusting the pH to 7.2-8.5, wherein the gel solution is subjected to self-crosslinking to obtain a hydrogel; or
wherein the polysaccharide derivative is a chitin/chitosan derivative, and in step (5), the thiolated chitin/chitosan derivative is dissolved in an aqueous solvent to prepare a gel solution with a concentration of 2-10%, adjusting the pH to 7.0-8.5, wherein the gel solution is subjected to self-crosslinking to obtain a hydrogel.

Method for preparing the above hydrogel, wherein the molar ratio of the hyaluronic acid derivative to EDC, NHS and the thiolating agent is 1 : 1-6 : 1-6 : 1-5; or the molar ratio of the chitin/chitosan derivative to EDC, NHS and the thiolating agent is 1 : 1-6 : 1-6 : 1-6.

Method for preparing the above hydrogel, wherein the thiolating agent is selected from the group consisting of cysteine and its hydrochloride salt, mercaptoethylamine and its hydrochloride salt, mercaptoacetic acid, mercaptopropionic acid and mercaptoethanol.

Also claimed is an intraocular vitreous substitute comprising the hydrogel as described above.

Also claimed is a thiolated polysaccharide derivative for use as an intraocular vitreous substitute, selected from:
(i) a thiolated hyaluronic acid derivative having the thiol substitution degree of 3% to 20%, wherein the hyaluronic acid derivative is selected from the group consisting of hydroxyethyl hyaluronic acid, hydroxypropyl hyaluronic acid, acetyl hyaluronic acid, butyryl hyaluronic acid, aminoacetic hyaluronic acid, aminopropionic hyaluronic acid, aminobutyric hyaluronic acid, hydrazide and hyaluronic acid; and
(ii) a thiolated chitin/chitosan derivative having the thiol substitution degree of 3% to 25%, wherein the chitin/chitosan derivative is selected from the group consisting of carboxyethyl chitosan, hydroxyethyl chitosan, hydroxypropyl chitosan, hydroxybutyl chitosan, succinyl chitosan, aminoacetic chitosan, aminopropionic chitosan, aminobutyric chitosan, hydroxyethyl succinyl chitosan, hydroxypropyl succinyl chitosan, hydroxybutyl succinyl chitosan, carboxymethyl chitin, and carboxyethyl chitin.

A method for preparing the thiolated polysaccharide derivative as described above, ( not according to the claimed invention) comprising the following steps:
(1) dissolving a polysaccharide derivative in water or DMSO solvent with stirring;
(2) adding carboxy-activating agents 1-(3-dimethylaminopropyl)-3-ethylcarbodiimide hydrochloride (EDC) and N-hydroxy succinimide (NHS) to the polysaccharide derivative solution to form a reaction system, adjusting the pH of the reaction system to 4-6, and stirring it for 0.5 to 3 h;
(3) adding a thiolating agent to the reaction system of step (2), adjusting the pH of the reaction system to 3.5 - 6, stirring it under dark condition to obtain a reaction product; and
(4) performing dialysis of the reaction product in acidic aqueous solution under dark condition, and freeze-drying the resulting solution; or precipitating the reaction product with ethanol, washing, precipitating with ethanol, dehydrating, and drying under vacuum; to obtain the thiolated polysaccharide derivative.

The thiolating agent is selected from the group consisting of cysteine and its hydrochloride salt, mercaptoethylamine and its hydrochloride salt, mercaptoacetic acid, mercaptopropionic acid and mercaptoethanol.

The molar ratio of the hyaluronic acid derivative to EDC, NHS, and the thiolating agent is 1 : 1-6 : 1-6 : 1-5; or the molar ratio of the chitin/chitosan derivative to EDC, NHS, and the thiolating agent is 1 : 1-6 : 1-6 : 1-6.

### BRIEF DESCRIPTION OF THE DRAWINGS

Figure 1 is a histological observation of the cornea of the operated eye stained with HE.
Figure 2 is a histological observation of the retina of the operated eye stained with HE.
Figure 3 is an OCT examination image of a normal rabbit eye (A) and an operated eye (B) on day 90 after surgery.

### DETAILED DESCRIPTION OF THE INVENTION

### Definitions

The terms "thiolated hyaluronic acid polysaccharide hydrogel", "thiolated hyaluronic acid derivative hydrogel", and "thiolated hyaluronic acid polysaccharide derivative hydrogel" can be used interchangeably in this invention, referring thiolated hyaluronic acid derivatives with grafted side chain groups

### Thiolated polysaccharide derivatives

In an embodiment, the invention relates to a thiolated hyaluronic acid derivative having a thiol substitution degree of 3% to 20%, wherein the hyaluronic acid derivative is selected from the group consisting of hydroxyethyl hyaluronic acid, hydroxypropyl hyaluronic acid, acetyl hyaluronic acid, butyryl hyaluronic acid, aminoacetic hyaluronic acid, aminopropionic hyaluronic acid, aminobutyric hyaluronic acid and hydrazide hyaluronic acid.

In another embodiment, the invention relates to a thiolated chitin/chitosan derivative having a thiol substitution degree of 3% to 25%, wherein the chitin/chitosan derivative is selected from the group consisting of carboxyethyl chitosan, hydroxyethyl chitosan, hydroxypropyl chitosan, hydroxybutyl chitosan, succinyl chitosan, aminoacetic chitosan, aminopropionic chitosan, aminobutyric chitosan, hydroxyethyl succinyl chitosan, hydroxypropyl succinyl chitosan, hydroxybutyl succinyl chitosan and carboxyethyl chitin.

### Method for preparing thiolated polysaccharide derivatives

In one aspect, there is described a method for preparing a thiolated polysaccharide derivative as described above. The method comprises the following steps:
(1) dissolving a polysaccharide derivative in water or DMSO solvent with stirring;
(2) adding carboxy-activating agents 1-(3-dimethylaminopropyl)-3-ethylcarbodiimide hydrochloride (EDC) and N-hydroxy succinimide (NHS) to the polysaccharide derivative solution to form a reaction system, adjusting the pH of the reaction system to 4-6, and stirring it for 0.5 to 3 h;
(3) adding a thiolating agent to the reaction system of step (2), adjusting the pH of the reaction system to 3.5 - 6, stirring it under dark condition to obtain a reaction product; and
(4) performing dialysis of the reaction product in acidic aqueous solution under dark condition, and freeze-drying the resulting solution; or precipitating the reaction product with ethanol, washing, precipitating with ethanol, dehydrating, and drying under vacuum; to obtain the thiolated polysaccharide derivative.

The thiolating agent is selected from the group consisting of cysteine and its hydrochloride salt, mercaptoethylamine and its hydrochloride salt, mercaptoacetic acid, mercaptopropionic acid and mercaptoethanol.

In an embodiment, the polysaccharide derivative according to the invention is a hyaluronic acid derivative, wherein the molar ratio of the hyaluronic acid derivative to EDC, NHS, and the thiolating agent is 1 : 1-6 : 1-6 : 1-5.

In an embodiment, the polysaccharide derivative according to the invention is a chitin/chitosan derivative, wherein the molar ratio of the chitin/chitosan derivative to EDC, NHS, and the thiolating agent is 1 : 1-6 : 1-6 : 1-6.

### Method for preparing hydrogels with thiolated polysaccharide derivatives

In one aspect, the invention relates to a method for preparing a hydrogel with a thiolated polysaccharide derivative as described above.

The aqueous solvent according to the invention is selected from the group consisting of water, physiological saline, phosphate buffer, physiological balanced solution and glucose solution.

In an embodiment, the thiolated polysaccharide derivative according to the invention is a thiolated hyaluronic acid derivative, wherein the method comprises the following steps: dissolving a thiolated hyaluronic acid derivative in an aqueous solvent to prepare a gel solution with a concentration of 2.5-6%, adjusting the pH to 7.2-8.5, and the gel solution is subjected to self-crosslinking to obtain a hydrogel.

In an embodiment, the thiolated polysaccharide derivative according to the invention is a thiolated chitin/chitosan derivative, wherein the method comprises the following steps: dissolving a thiolated chitin/chitosan derivative in an aqueous solvent to prepare a gel solution with a concentration of 2-10%, adjusting the pH to 7.0-8.5, and the gel solution is subjected to self-crosslinking to obtain a hydrogel.

### Use of thiolated polysaccharide derivatives (not according to the claimed invention)

In one aspect, the invention relates to a use of a thiolated polysaccharide derivative as described above in manufacture of an intraocular vitreous substitute.

In one aspect, the present invention relates to the use of the aforementioned thiolated polysaccharide derivatives for preparing intraocular vitreous substitutes, wherein the thiolated polysaccharide derivatives according to the invention form a hydrogel with an aqueous solvent, and the hydrogel as formed is used as an intraocular vitreous substitute.

In an embodiment, the present invention relates to the use of thiolated hyaluronic acid derivatives for preparing intraocular vitreous substitutes.

In an embodiment, the present invention relates to the use of thiolated chitin/chitosan derivatives for preparing intraocular vitreous substitutes.

### Hydrogels

In one aspect, the invention relates to a thiolated polysaccharide derivative hydrogel selected from:
(i) a thiolated hyaluronic acid polysaccharide hydrogel, which is a polysaccharide hydrogel formed from one or two of thiolated hyaluronic acid derivatives and an aqueous solvent. The mass percentage concentration of the thiolated hyaluronic acid derivative is 3 to 6%, and the thiol substitution degree of the thiolated hyaluronic acid derivative is 3 to 20%. The thiolated hyaluronic acid derivative is prepared by thiolation reaction using hydroxyethyl hyaluronic acid, hydroxypropyl hyaluronic acid, acetyl hyaluronic acid, butyryl hyaluronic acid, aminoacetic hyaluronic acid, aminopropionic hyaluronic acid, aminobutyric hyaluronic acid, or hydrazide hyaluronic acid; and
(ii) a thiolated chitin/chitosan derivative hydrogel, which is a polysaccharide hydrogel formed from one or two of thiolated chitin/chitosan derivatives and an aqueous solvent. The mass percentage concentration of the thiolated chitin/chitosan derivative is 2-10%, and the thiol substitution degree of the thiolated chitin/chitosan derivative is 3-25%. The thiolated chitin/chitosan derivative is prepared by thiolation reaction using carboxyethyl chitosan, hydroxyethyl chitosan, hydroxypropyl chitosan, hydroxybutyl chitosan, succinyl chitosan, aminoacetic chitosan, aminopropionic chitosan, aminobutyric chitosan, hydroxyethyl succinyl chitosan, hydroxypropyl succinyl chitosan, hydroxybutyl succinyl chitosan, carboxymethyl chitin or carboxyethyl chitin.

The mass percentage concentration of the thiolated hyaluronic acid derivative is 3-6%.

### Methods

In one aspect, there is disclosed a method for preparing a thiolated polysaccharide derivative hydrogel as described above. The method comprises the following steps:
(1) dissolving a polysaccharide derivative in water or DMSO solvent with stirring;
(2) adding carboxy-activating agents 1-(3-dimethylaminopropyl)-3-ethylcarbodiimide hydrochloride (EDC) and N-hydroxy succinimide (NHS) to the polysaccharide derivative solution to form a reaction system, adjusting the pH of the reaction system to 4-6, and stirring it for 0.5 to 3 h, followed by adding a thiolating agent to the reaction system, adjusting the pH of the reaction system to 3.5 - 6, and stirring it under dark condition;
(3) performing dialysis of the reaction product of step (2) in acidic aqueous solution under dark condition and freeze-drying the resulting solution; or precipitating the reaction product of step (2) with ethanol, washing, precipitating with ethanol, dehydrating, and drying under vacuum; to obtain a thiolated polysaccharide derivative, which is stored with sealing under dark condition at low temperature; and
(4) dissolving the thiolated polysaccharide derivative in an aqueous solvent to prepare a gel solution with a concentration of 2-10%, adjusting the pH to 7.0-8.5, wherein the gel solution is subjected to self-crosslinking to obtain a hydrogel.

The thiolating agent used in step (2) is selected from the group consisting of cysteine and its hydrochloride salt, mercaptoethylamine and its hydrochloride salt, mercaptoacetic acid, mercaptopropionic acid and mercaptoethanol.

The aqueous solvent used in step (4) is selected from the group consisting of water, physiological saline, phosphate buffer, physiological balanced solution and glucose solution.

In an embodiment, the thiolated polysaccharide derivative hydrogel is a thiolated hyaluronic acid polysaccharide hydrogel, and the polysaccharide derivative is a hyaluronic acid derivative, wherein in step (2), the molar ratio of the hyaluronic acid derivative to EDC, NHS and the thiolating agent is 1 : 1-6 : 1-6 : 1-5, and in step (4), the prepared thiolated hyaluronic acid derivative is dissolved in an aqueous solvent to prepare a gel solution with a concentration of 3-6% adjusting the pH to 7.2-8.5, wherein the gel solution is subjected to self-crosslinking to obtain a thiolated hyaluronic acid polysaccharide hydrogel. The concentration of the gel solution prepared by dissolving the thiolated hyaluronic acid derivative in an aqueous solvent is 3-6%.

In an embodiment, the thiolated polysaccharide derivative hydrogel is a thiolated chitin/chitosan derivative hydrogel, and the polysaccharide derivative is a chitin/chitosan derivative, wherein in step (2), the molar ratio of the chitin/chitosan derivative to EDC, NHS and the thiolating agent is 1 : 1-6 : 1-6 : 1-6, and in step (4), the prepared thiolated chitin/chitosan derivative is dissolved in an aqueous solvent to prepare a gel solution with a concentration of 2-10%, adjusting the pH to 7.0-8.5, wherein the gel solution is subjected to self-crosslinking to obtain a thiolated chitin/chitosan derivative hydrogel.

### Use of the hydrogels (not according to the claimed invention)

In one aspect, the present invention relates to the use of the above-mentioned thiolated polysaccharide derivatives hydrogel for preparing intraocular vitreous substitutes, preferably to the use of thiolated hyaluronic acid polysaccharide hydrogel or thiolated chitosan/chitosan derivatives hydrogel for preparing intraocular vitreous substitutes.

### Intraocular vitreous substitutes

In one aspect, the present invention relates to an intraocular vitreous substitute, which comprises a hydrogel formed from a thiolated polysaccharide derivative and an aqueous solvent. The intraocular vitreous substitute of the present invention comprises a thiolated polysaccharide derivative hydrogel, wherein the thiolated polysaccharide derivative hydrogel is selected from:
(i) a thiolated hyaluronic acid polysaccharide derivative hydrogel, which is a polysaccharide hydrogel formed from one or two of thiolated hyaluronic acid derivatives and an aqueous solvent, wherein the mass percentage concentration of the thiolated hyaluronic acid derivative is 3% to 6%, and the thiol substitution degree of the thiolated hyaluronic acid derivative is 3% to 20%; the hyaluronic acid derivative is selected from the group consisting of hydroxyethyl hyaluronic acid, hydroxypropyl hyaluronic acid, acetyl hyaluronic acid, butyryl hyaluronic acid, aminoacetic hyaluronic acid, aminopropionic hyaluronic acid, aminobutyric hyaluronic acid, and hydrazide hyaluronic acid; and
(ii) a thiolated chitin/chitosan derivative hydrogel, which is a polysaccharide hydrogel formed from one or two of thiolated chitin/chitosan derivatives and an aqueous solvent, wherein the mass percentage concentration of the thiolated chitin/chitosan derivative is 2% to 10%, and the thiol substitution degree of the thiolated chitin/chitosan derivative is 3% to 25%; the chitin/chitosan derivative is selected from the group consisting of carboxyethyl chitosan, hydroxyethyl chitosan, hydroxypropyl chitosan, hydroxybutyl chitosan, succinyl chitosan, aminoacetic chitosan, aminopropionic chitosan, aminobutyric chitosan, hydroxyethyl succinyl chitosan, hydroxypropyl succinyl chitosan, hydroxybutyl succinyl chitosan, carboxymethyl chitin, and carboxyethyl chitin.

The mass percentage concentration of the thiolated hyaluronic acid derivative is 3-6%.

### Thiolated polysaccharide derivative hydrogels for use in the manufacture of intraocular vitreous substitutes

The inventive thiolated polysaccharide derivative hydrogel for use in the manufacture of intraocular vitreous substitutes is selected from:
(i) a thiolated hyaluronic acid polysaccharide derivative hydrogel, which is a polysaccharide hydrogel formed from one or two of thiolated hyaluronic acid derivatives and an aqueous solvent. The mass percentage concentration of the thiolated hyaluronic acid derivative is 3 to 6%, and the thiol substitution degree of the thiolated hyaluronic acid derivative is 3 to 20%. The hyaluronic acid derivative is selected from the group consisting of hydroxyethyl hyaluronic acid, hydroxypropyl hyaluronic acid, acetyl hyaluronic acid, butyryl hyaluronic acid, aminoacetic hyaluronic acid, aminopropionic hyaluronic acid, aminobutyric hyaluronic acid, and hydrazide hyaluronic acid; and
(ii) a thiolated chitin/chitosan derivative hydrogel, which is a polysaccharide hydrogel formed from one or two of thiolated chitin/chitosan derivatives and an aqueous solvent. The mass percentage concentration of the thiolated chitin/chitosan derivative is 2-10%, and the thiol substitution degree of the thiolated chitin/chitosan derivative is 3-25%. The chitin/chitosan derivative is selected from the group consisting of carboxyethyl chitosan, hydroxyethyl chitosan, hydroxypropyl chitosan, hydroxybutyl chitosan, succinyl chitosan, aminoacetic chitosan, aminopropionic chitosan, aminobutyric chitosan, hydroxyethyl succinyl chitosan, hydroxypropyl succinyl chitosan, hydroxybutyl succinyl chitosan, carboxymethyl chitin, and carboxyethyl chitin.

The mass percentage concentration of the thiolated hyaluronic acid derivative is 3-6%.

In another aspect, the invention relates to a method for preparing the afore-mentioned thiolated polysaccharide derivative hydrogel for use in the manufacture of intraocular vitreous substitutes. The method comprises the following steps:
(1) dissolving a polysaccharide derivative in water or DMSO solvent with stirring;
(2) adding carboxy-activating agents 1-(3-dimethylaminopropyl)-3-ethylcarbodiimide hydrochloride (EDC) and N-hydroxy succinimide (NHS) to the polysaccharide derivative solution to form a reaction system, adjusting the pH of the reaction system to 4-6, and stirring it for 0.5 to 3 h, followed by adding a thiolating agent to the reaction system, adjusting the pH of the reaction system to 3.5 - 6, and stirring it under dark condition;
(3) performing dialysis of the reaction product of step (2) in acidic aqueous solution under dark condition and freeze-drying the resulting solution; or precipitating the reaction product of step (2) with ethanol, washing, precipitating with ethanol, dehydrating, and drying under vacuum; to obtain a thiolated polysaccharide derivative, which is stored with sealing under dark condition at low temperature; and
(4) dissolving the thiolated polysaccharide derivative in an aqueous solvent to prepare a gel solution with a concentration of 2-10%, adjusting the pH to 7.0-8.5, wherein the gel solution is subjected to self-crosslinking to obtain a thiolated polysaccharide derivative hydrogel for use in the manufacture of intraocular vitreous substitutes.

The thiolating agent used in step (2) is selected from the group consisting of cysteine and its hydrochloride salt, mercaptoethylamine and its hydrochloride salt, mercaptoacetic acid, mercaptopropionic acid and mercaptoethanol.

The aqueous solvent used in step (4) is selected from the group consisting of water, physiological saline, phosphate buffer, physiological balanced solution and glucose solution.

In an embodiment, the thiolated polysaccharide derivative hydrogel is a thiolated hyaluronic acid polysaccharide hydrogel, and the polysaccharide derivative is a hyaluronic acid derivative. In step (2), the molar ratio of the hyaluronic acid derivative to EDC, NHS and the thiolating agent is 1 : 1-6 : 1-6 : 1-5, and in step (4), the prepared thiolated hyaluronic acid derivative is dissolved in an aqueous solvent to prepare a gel solution with a concentration of 3-6%, adjusting the pH to 7.2-8.5, wherein the gel solution is subjected to self-crosslinking to obtain a thiolated hyaluronic acid polysaccharide hydrogel.

The concentration of the gel solution prepared by dissolving the thiolated hyaluronic acid derivative in an aqueous solvent is 3-6%.

In an embodiment, the thiolated polysaccharide derivative hydrogel is a thiolated chitin/chitosan derivative hydrogel, and the polysaccharide derivative is a chitin/chitosan derivative. In step (2), the molar ratio of the chitin/chitosan derivative to EDC, NHS and the thiolating agent is 1 : 1-6 : 1-6 : 1-6, and in step (4), the prepared thiolated chitin/chitosan derivative is dissolved in an aqueous solvent to prepare a gel solution with a concentration of 2-10%, adjusting the pH to 7.0-8.5, wherein the gel solution is subjected to self-crosslinking to obtain a thiolated chitin/chitosan derivative hydrogel.

### Thiolated hyaluronic acid polysaccharide hydrogel and preparation method therefor and use thereof

A thiolated hyaluronic acid polysaccharide hydrogel, characterized in that the polysaccharide hydrogel is formed from one or two of thiolated hyaluronic acid derivatives and an aqueous solvent. The mass percentage concentration of the thiolated hyaluronic acid derivative is 3-6%, and the thiol substitution degree thereof is 3-20%. The thiolated hyaluronic acid derivative is prepared by thiolating reaction of hydroxyethyl hyaluronic acid, hydroxypropyl hyaluronic acid, acetyl hyaluronic acid, butyryl hyaluronic acid, aminoacetic hyaluronic acid, aminopropionic hyaluronic acid, aminobutyric hyaluronic acid, or hydrazide hyaluronic acid.

The preparation method of the aforementioned thiolated hyaluronic acid polysaccharide hydrogel includes the following steps:
(1) dissolving a hyaluronic acid derivative in water or DMSO solvent with stirring;
(2) adding carboxy-activating agents 1-(3-dimethylaminopropyl)-3-ethylcarbodiimide hydrochloride (EDC) and N-hydroxy succinimide (NHS) to the hyaluronic acid derivative solution, adjusting the pH of the reaction system to 4-6, and stirring it for 0.5 to 3 h, then adding a thiolating agent to the reaction system, adjusting the pH of the reaction system to 3.5 - 6, stirring it under dark condition;
(3) performing dialysis of the reaction product in acidic aqueous solution under dark condition and freeze-drying the resulting solution; or precipitating the reaction product with ethanol, washing, precipitating with ethanol, dehydrating, and drying under vacuum; to obtain a thiolated hyaluronic acid derivative, which is stored with sealing under dark condition at low temperature;
(4) dissolving the thiolated hyaluronic acid derivative in an aqueous solvent to prepare a gel solution with a concentration of 3-6%, adjusting the pH to 7.2-8.5, wherein the gel solution is subjected to self-crosslinking to obtain a thiolated hyaluronic acid polysaccharide hydrogel.

In step (2), the molar ratio of the hyaluronic acid derivative to 1-(3-dimethylaminopropyl)-3-ethylcarbodiimide hydrochloride, N-hydroxysuccinimide, and the thiolating agent is 1: 1-6 : 1-6 : 1-5.

In step (2), the thiolating agent is selected from the group consisting of cysteine and its hydrochloride salt, mercaptoethylamine and its hydrochloride salt, mercaptoacetic acid, mercaptopropionic acid and mercaptoethanol.

In step (4), the aqueous solvent is selected from the group consisting of water, physiological saline, phosphate buffer, physiological balanced solution and glucose solution.

The above-mentioned thiolated hyaluronic acid polysaccharide hydrogel is used for preparing intraocular vitreous substitutes.

### Thiolated chitin/chitosan polysaccharide hydrogel and preparation method therefor and use thereof

A thiolated chitin/chitosan polysaccharide hydrogel, characterized in that the polysaccharide hydrogel is formed from one or two of thiolated chitin/chitosan derivatives and an aqueous solvent. The mass percentage concentration of the thiolated chitin/chitosan derivative is 2-10%, and the thiol substitution degree thereof is 3-25%. The thiolated chitin/chitosan derivative is prepared by thiolating reaction of carboxyethyl chitosan, hydroxyethyl chitosan, hydroxypropyl chitosan, hydroxybutyl chitosan, succinyl chitosan, aminoacetic chitosan, aminopropionic chitosan, aminobutyric chitosan, hydroxyethyl succinyl chitosan, hydroxypropyl succinyl chitosan, hydroxybutyl succinyl chitosan, carboxymethyl chitin, or carboxyethyl chitin.

The preparation method of the aforementioned thiolated chitin/chitosan polysaccharide hydrogel includes the following steps:
(1) dissolving a chitin/chitosan derivative in water or DMSO solvent with stirring;
(2) adding carboxy-activating agents 1-(3-dimethylaminopropyl)-3-ethylcarbodiimide hydrochloride (EDC) and N-hydroxy succinimide (NHS) to the hyaluronic acid derivative solution, adjusting the pH of the reaction system to 4-6, and stirring it for 0.5 to 3 h, then adding a thiolating agent to the reaction system, adjusting the pH of the reaction system to 3.5 - 6, stirring it under dark condition;
(3) performing dialysis of the reaction product in acidic aqueous solution under dark condition and freeze-drying the resulting solution; or precipitating the reaction product with ethanol, washing, precipitating with ethanol, dehydrating, and drying under vacuum; to obtain a thiolated hyaluronic acid derivative, which is stored with sealing under dark condition at low temperature;
(4) dissolving the thiolated chitin/chitosan derivative in an aqueous solvent to prepare a gel solution with a concentration of 2-10%, adjusting the pH to 7.0-8.5, wherein the gel solution is subjected to self-crosslinking to obtain a thiolated chitin/chitosan polysaccharide hydrogel.

In step (2), the molar ratio of the chitin/chitosan derivative to 1-(3-dimethylaminopropyl)-3-ethylcarbodiimide hydrochloride, N-hydroxysuccinimide, and the thiolating agent is 1: 1-6 : 1-6 : 1-5.

In step (2), the thiolating agent is selected from the group consisting of cysteine and its hydrochloride salt, mercaptoethylamine and its hydrochloride salt, mercaptoacetic acid, mercaptopropionic acid and mercaptoethanol.

In step (4), the aqueous solvent is selected from the group consisting of water, physiological saline, phosphate buffer, physiological balanced solution and glucose solution.

The above-mentioned thiolated chitin/chitosan polysaccharide hydrogel is used for preparing intraocular vitreous substitutes.

### Advantages of the present invention

The thiolated hyaluronic acid derivatives hydrogel according to the present invention were prepared by grafting side chains onto the hydroxyl and carboxyl group of the hyaluronic acid and controlling a moderate thiol substitution degree. The intermolecular and intramolecular support of the thiolated hyaluronic acid hydrogel is increased, allowing the hydrogel to have both softness and good resilience, maintaining its gel-like form, and meeting the performance requirements for vitreous substitutes. Additionally, the introduction of side chain groups to the hyaluronic acid derivatives reduces their binding affinity to biological enzymes in the body, thereby improving the hydrogel's resistance to degradation.

The thiolated chitin/chitosan derivatives hydrogel prepared by the present invention uses chitin/chitosan derivatives with side chains connected by β-1,4-glycosidic bonds as raw materials. A moderate thiol substitution degree is controlled, and the thiol groups undergo self-crosslinking via disulfide bonds to form the hydrogel. On one hand, the β-1,4-glycosidic bonds in chitin/chitosan derivatives enhance the hydrogel's resistance to degradation. On the other hand, the side chain groups of chitin/chitosan derivatives enhance the intermolecular and intramolecular support of the hydrogel, increasing its softness and good resilience. Furthermore, the stability of the hydrogel's disulfide bonds is good, and an appropriate thiol substitution degree helps maintain the stability of the hydrogel's form.

The hydrogel of the present invention, as a vitreous substitute, can improve shock absorption. Its excellent self-healing function and resilience can maintain the shape of the eyeball and keep the retina in position.

The following detailed description of the present invention is further illustrated with reference to the drawings and specific embodiments. The specific embodiments provided are only intended to explain the technical solution of the present invention and are not intended to limit the scope of the present invention.

### EXAMPLES

### Example 1: Preparation of thiolated hydroxypropyl hyaluronic acid derivative hydrogel (SH-HP-HA)

2.3g of hydroxypropyl hyaluronic acid (HP-HA) was weighed and dissolved in 100ml of dimethyl sulfoxide (DMSO) with stirring, forming a solution with a concentration of 2.3%. Then, 5.76g of 1-(3-dimethylaminopropyl)-3-ethylcarbodiimide hydrochloride (EDC) and 3.45g of N-hydroxysuccinimide (NHS) were added and dissolved with stirring. The pH was adjusted to 4.5 using 0.2mol/L NaOH and 0.2mol/L HCl solutions, and the mixture was stirred at room temperature under dark condition for 2 hours. 2.83g of cysteamine hydrochloride was weighed and added to the reaction system, and the pH was adjusted to 4.5. The reaction was continued under dark condition for 24 hours. After the reaction, the reaction product was precipitated with ethanol, dissolved in water, precipitated with ethanol again, dehydrated with ethanol, and dried under vacuum at 45°C, yielding a thiolated hydroxypropyl hyaluronic acid (SH-HP-HA). The product was stored with sealing at 4-10°C under dark condition. The molar ratio of HP-HA:EDC:NHS:cysteamine hydrochloride was 1:6:6:5 in this reaction, and the thiolation rate measured by Ellman's colorimetric method was 18.8%.

0.25g of the prepared thiolated hydroxypropyl hyaluronic acid was weighed and placed in a clean beaker. 10ml of phosphate buffer (0.2mol/L) with a pH of 7.6 was then added, and the mixture was stirred at room temperature until dissolved, resulting in a gel solution with a mass percentage concentration of 2.5%. The mixture was left for 35 minutes and was subjected to self-crosslinking to form the thiolated hydroxypropyl hyaluronic acid derivative hydrogel, referred to as SH-HP-HA hydrogel.

### Example 2: Preparation of thiolated hydroxyethyl hyaluronic acid derivative hydrogel (SH-HE-HA)

2.35g of hydroxyethyl hyaluronic acid (HE-HA) was weighed and dissolved in 100ml of deionized water with stirring, forming a 2.4% solution. Then, 3.84g of EDC and 2.3g of NHS were added and dissolved with stirring. The pH was adjusted to 4.5, and the mixture was stirred at room temperature under dark condition for 2 hours. 2.36g of cysteine hydrochloride was weighted and added it to the reaction system, and the pH was adjusted to 4.5. The reaction was continued under dark condition for 24 hours. After the reaction, transferred the reaction mixture into a dialysis bag and dialyzed it with acidic water solution at a pH of 4.5 under dark condition. The dialyzed product was then freeze-dried to obtain mercaptoethyl hyaluronic acid (SH-HE-HA), which was stored in a sealed container at low temperature under dark condition. The molar ratio of HE-HA:EDC:NHS:cysteine hydrochloride was 1:4:4:3, and the measured thiolation rate was 12.26%.

0.35g of the prepared thiolated hydroxyethyl hyaluronic acid was weighed and placed in a clean beaker. 10ml of phosphate buffer (0.2mol/L) with a pH of 7.8 was then added into the mixure, and the mixture was stirred at room temperature until dissolved, resulting in a gel solution with a mass percentage concentration of 3.5%. The mixture was left for 15 minutes and was subjected to self-crosslinking to form the thiolated hydroxyethyl hyaluronic acid derivative hydrogel, referred to as SH-HE-HA hydrogel.

### Example 3: Preparation of thiolated aminobutyric hyaluronic acid derivative hydrogel (SH-AB-HA)

2.44g of aminobutyric hyaluronic acid (AB-HA) was weighed and dissolved in 100ml of deionized water with stirring, forming a 2.4% solution. Then, 1.92g of EDC and 1.15g of NHS were added and dissolved with stirring. The pH was adjusted to 5.5, and the mixture was stirred at room temperature under dark condition for 1 hour. 1.57g of cysteine hydrochloride was weighed and added to the reaction system, and the pH was adjusted to 5.0. The reaction was continued under dark condition for 24 hours. After the reaction, the reaction mixture was placed into a dialysis bag and dialyzed against acidic water with a pH of 4.5 under dark condition. The dialyzed product was then freeze-dried to obtain thiolated aminobutyric hyaluronic acid (SH-AB-HA), which was stored at low temperature under dark condition. The molar ratio of AB-HA : EDC : NHS : cysteine hydrochloride was 1:2:2:2, and the measured thiolation rate was 7.58%.

0.45g of the prepared thiolated aminobutyric hyaluronic acid was weighed and placed in a clean beaker. Then, 10ml of deionized water was added, and the mixture was stirred at room temperature until dissolved. The pH was adjusted to 7.4-7.8 using NaOH (0.1mol/L) solution, resulting in a gel solution with a mass percentage concentration of 4.5%. The mixture was left for 14 minutes and was subjected to self-crosslinking to form the thiolated aminobutyric hyaluronic acid derivative hydrogel, referred to as SH-AB-HA hydrogel.

### Example 4: Preparation of thiolated carboxylic hydrazide hyaluronic acid (SH-CH-HA) hydrogel

2.38g of carboxylic hydrazide hyaluronic acid (CH-HA) was weighed and dissolved in 100ml of deionized water with stirring, forming a 2.4% solution. Then, 0.96g of EDC and 0.57g of NHS were added and dissolved with stirring. The pH was adjusted to 4.5, and the mixture was stirred at room temperature under dark condition for 2 hours. Then, 0.57g of cysteamine hydrochloride was weighed and added to the reaction system, and the pH was adjusted to 4.5. The reaction was continued under dark condition for 24 hours. After the reaction, the reaction mixture was transferred into a dialysis bag and dialyzed against acidic water solution with a pH of 4.5 under dark condition. After dialysis, the product was freeze-dried at low temperature to obtain thiolated carboxylic hydrazide hyaluronic acid (SH-CH-HA), which was sealed and stored at low temperature under dark condition. The molar ratio of CH-HA : EDC : NHS : cysteamine hydrochloride in this reaction was 1:1:1:1, and the measured thiolation rate was 4.68%.

0.55g of the prepared thiolated carboxylic hydrazide hyaluronic acid was weighed and placed in a clean beaker. 10ml of deionized water was added, and the mixture was stirred at room temperature until dissolved. The pH was adjusted to 7.8-8.0 using NaOH (0.1mol/L) solution, resulting in a gel solution with a mass percentage concentration of 5.5%. The mixture was left for 14 minutes and was subjected to self-crosslinking to form the thiolated carboxylic hydrazide hyaluronic acid derivative hydrogel, referred to as SH-CH-HA hydrogel.

### Example 5: Preparation of thiolated succinyl chitosan hydrogel (SH-SU-CTS)

2.6g of succinyl chitosan (SU-CTS) was weighed and dissolved in 100ml of deionized water with stirring, forming a 2.6% solution. Then, 11.5g of 1-(3-dimethylaminopropyl)-3-ethylcarbodiimide hydrochloride (EDC) and 6.9g of N-hydroxysuccinimide (NHS) were added and dissolved with stirring. The pH was adjusted to 4.5 using 0.2mol/L NaOH and 0.2mol/L HCl solutions, and the mixture was stirred at room temperature under dark condition for 2 hours. Then, 6.8g of cysteamine hydrochloride was weighed and added to the reaction system, and the pH was adjusted to 4.5. The reaction was continued under dark condition for 10 hours. After the reaction, the product was precipitated with ethanol, dissolved in water, precipitated with ethanol again, dehydrated with ethanol, and dried under vacuum at 45°C, yielding a thiolated succinyl chitosan (SH-SU-CTS). The product was sealed and stored at 4-10°C under dark condition. The molar ratio of SU-CTS : EDC : NHS : cysteamine hydrochloride in this reaction was 1:6:6:6, and the thiolation rate measured by Ellman's colorimetric method was 23.8%.

0.25g of the prepared thiolated succinyl chitosan was weighed and placed in a clean beaker. 10ml of phosphate buffer solution (0.2mol/L) with a pH of 7.6 was then added, and the mixture was stirred at room temperature until dissolved, resulting in a gel solution with a mass percentage concentration of 2.5%. The mixture was left for 23 minutes and was subjected to self-crosslinking to form the thiolated succinyl chitosan hydrogel, referred to as SH-SU-CTS hydrogel.

### Example 6: Preparation of thiolated hydroxypropyl chitosan hydrogel (SH-HP-CTS)

2.2g of hydroxypropyl chitosan (HP-CTS) was weighed and stirred in 100ml of dimethyl sulfoxide (DMSO) with stirring, forming a 2.2% solution. Then, 7.68g of EDC and 4.6g of NHS were added and dissolved with stirring. The pH was adjusted to 4.5, and the mixture was stirred under dark condition at room temperature for 2 hours. 4.73g of cysteine hydrochloride was weighed and added to the reaction system. The pH was adjusted to 4.5, and the reaction was continued under dark condition for 16 hours. After the reaction, the reaction mixture was loaded into a dialysis bag and dialyzed against acidic water with a pH of 4.5 under dark condition. The dialyzed product was freeze-dried to obtain thiolated hydroxypropyl chitosan (SH-HP-CTS), which was stored in a low-temperature under dark condition. The molar ratio of HP-CTS : EDC : NHS : cysteine hydrochloride was 1:4:4:3, and the measured thiolation rate was 15.6%.

0.4g of the prepared thiolated hydroxypropyl chitosan was weighed and placed in a clean beaker.

10ml of phosphate buffer solution (0.2mol/L) with a pH of 7.8 was added and the mixture was stirred at room temperature until dissolved, resulting in a gel solution with a mass percentage concentration of 4%. The mixture was left for 11 minutes and was subjected to self-crosslinking to form the thiolated hydroxypropyl chitosan hydrogel, referred to as SH-HP-CTS hydrogel.

### Example 7: Preparation of thiolated carboxymethyl chitosan hydrogel (SH-CM-CT) ( not according to the claimed invention)

2.8g of carboxymethyl chitosan (CM-CT) was weighed and dissolved in 100ml of deionized water with stirring, forming a 2.8% solution. 5.76g of EDC and 3.45g of NHS were added and dissolved with stirring. The pH was adjusted to 5.5, and the mixture was stirred under dark condition at room temperature for 1 hour. Then, 3.15g of cysteine hydrochloride was weighed and added to the reaction system, and the pH was adjusted to 5.0. The reaction was continued under dark condition for 24 hours. After the reaction, the reaction mixture was loaded into dialysis bags and dialyzed against acidified water with a pH of 4.5 under dark condition. The dialyzed product was freeze-dried to obtain thiolated carboxymethyl chitosan (SH-CM-CT), which was stored sealed at low temperature under dark condition. The molar ratio of CM-CT : EDC : NHS : cysteine hydrochloride was 1:3:3:2, and the measured thiolation rate was 9.4%.

0.5g of the prepared thiolated carboxymethyl chitosan was weighed and placed in a clean beaker.

10ml of deionized water was added, and the mixture was stirred at room temperature until dissolved. The pH was adjusted to 7.8-8.0 using NaOH (0.1mol/L) solution, resulting in a gel solution with a mass percentage concentration of 5%. The mixture was left for 10 minutes and was subjected to self-crosslinking to form the thiolated carboxymethyl chitosan hydrogel, referred to as SH-CM-CT hydrogel.

### Example 8: Preparation of thiolated hydroxyethyl chitosan hydrogel (SH-HE-CTS)

3.1g of hydroxyethyl chitosan (HE-CTS) was weighed and dissolved in 100ml of deionized water with stirring, forming a 3.1% solution. Then, 2.88g of EDC and 1.73g of NHS were added and dissolved with stirring. The pH was adjusted to 4.5, and the mixture was stirred at room temperature under dark condition for 2 hours. Then, 1.7g of cysteamine hydrochloride was added to the reaction system, and the pH was adjusted to 4.5. The reaction was continued under dark condition for 24 hours. After the reaction, the reaction mixture was loaded into a dialysis bag and dialyzed against acidic water solution with a pH of 4.5 under dark condition. After dialysis, the product was freeze-dried to obtain thiolated hydroxyethyl chitosan (SH-HE-CTS). The product was then sealed and stored under low temperature under dark condition. The molar ratio of HE-CTS : EDC : NHS : cysteamine hydrochloride in this reaction was 1:1:1:1, and the measured thiolation rate was 3.8%.

0.85g of the prepared thiolated hydroxyethyl chitosan was weighed and placed in a clean beaker. 10ml of deionized water was added, and the mixture was stirred at room temperature until dissolved. The pH was adjusted to 7.8-8.0 using NaOH (0.1mol/L) solution, resulting in a gel solution with a mass percentage concentration of 8.5%. The mixture was left for 10 minutes and was subjected to self-crosslinking to form the thiolated hydroxyethyl chitosan hydrogel, referred to as SH-HE-CTS hydrogel.

### Comparative Example 1: Preparation of control thiolated hyaluronic acid hydrogel (SH-HA-1)

2.0g of hyaluronic acid (HA) was weighed and dissolved in 100ml of deionized water with stirring, forming a 2% solution. Then, 3.84g of EDC and 2.3g of NHS were added and dissolved with stirring. The pH was adjusted to 5.5 and the mixture was stirred under dark condition at room temperature for 2 hours. 2.4g of cysteine hydrochloride was weighed and added to the reaction system, and the pH was adjusted to 5.0. The reaction continued under dark condition for 24 hours. After the reaction, the reaction mixture was loaded into a dialysis bag and dialyzed against acidic water solution with a pH of 4.5 under dark condition. After dialysis, the product was freeze-dried to obtain thiolated hyaluronic acid (SH-HA-1), which was stored at low temperature under dark condition. The molar ratio of HA : EDC : NHS : cysteine hydrochloride was 1:4:4:3, and the measured thiolation rate was 12.48%.

0.35g of the prepared thiolated hyaluronic acid was weighed and placed in a clean beaker. 10ml of deionized water was added, and the mixture was stirred at room temperature until dissolved. The pH was adjusted to 7.4-7.8 using NaOH (0.1mol/L) solution, resulting in a gel solution with a mass percentage concentration of 3.5%. The mixture was left for 12 minutes and was subjected to self-crosslinking to form the control thiolated hyaluronic acid hydrogel, referred to as SH-HA-1 hydrogel.

### Comparative Example 2: Preparation of control thiolated hyaluronic acid hydrogel (SH-HA-2)

2.0g of hyaluronic acid (HA) was weighed and stirred to dissolve in 100ml of deionized water with stirring, forming a 2% solution. Then, 2.9g of EDC and 1.73g of NHS were added and dissolved with stirring. The pH was adjusted to 5.5 and the mixture was stirred under dark condition at room temperature for 2 hours. Then, 1.58g of hydrochloric acid salt of cysteine was weighed and added to the reaction system. The pH was adjusted to 5.0, and the reaction continued under dark conditions for 24 hours. After the reaction, the reaction mixture was loaded into a dialysis bag and dialyzed against acidic water solution with a pH of 4.5, under dark conditions. After dialysis, the product was freeze-dried to obtain thiolated hyaluronic acid (SH-HA-2), which was then sealed and stored at low temperature under dark condition. The molar ratio of HA : EDC : NHS : cysteine hydrochloric acid salt was 1:3:3:2, and the measured thiolation rate was 9.0%.

0.5g of the prepared thiolated hyaluronic acid was weighed and placed in a clean beaker. 10ml of deionized water was and the mixture was stirred at room temperature until dissolved. The pH was adjusted to 7.4-7.8 using NaOH (0.1mol/L) solution, resulting in a gel solution with a mass percentage concentration of 5%. The mixture was left for 10 minutes and was subjected to self-crosslinking to form the control thiolated hyaluronic acid hydrogel, referred to as SH-HA-2 hydrogel.

### Example 9: Transmittance measurement of the hydrogels

An appropriate amount of hydrogels prepared in Examples 1-8 and Comparative Examples 1-2 were respectively weighed into a phosphate buffer solution (pH 7.4) for swelling until equilibrium was reached, and then they were placed in glass color dishes. The absorbance values (A) were measured in the wavelength range of visible light (400-800 nm). The transmittance (%) of the hydrogels was calculated using the formula T(%) = 10^{2-A}. The results showed that the transmittance of the hydrogels was dependent on the wavelength and gel concentration. The transmittance of each hydrogel increased with increasing wavelength, and the trend was consistent. At a wavelength of 400 nm, the transmittance of hydrogels from Examples 1-4 and Comparative Example 1 was greater than 87%, while the transmittance of hydrogels from Examples 5-8 and Comparative Example 2 was greater than 85%. At wavelengths greater than 600 nm, the transmittance of all hydrogels was greater than 90%. The transmittance of low-concentration hydrogels was higher than that of high-concentration hydrogels.

### Example 10: Measurement of compressive performance of the hydrogels

Cylindrical samples with a diameter of 8 mm and a height of 10 mm were prepared using the hydrogels prepared in Examples 1-8 and Comparative Examples 1-2. The compressive performance of each hydrogel was measured using a universal material tester. The results showed that compared to the thiolated hyaluronic acid hydrogel in Comparative Examples 1-2, the fracture strain of the thiolated hyaluronic acid derivative hydrogel and thiolated chitin/chitosan derivative hydrogel prepared in Examples 1-8 were all greater than 70%. This was significantly higher than the control hydrogel, indicating that the thiolated hyaluronic acid derivative hydrogel and thiolated chitin/chitosan derivative hydrogel were more flexible than the thiolated hyaluronic acid hydrogel. Additionally, all the hydrogels exhibited good self-healing functionality and were able to recover their initial shape after compression. The results are shown in the table below.

**Table 1 shows the results of the compression performance tests on thiolated polysaccharide hydrogels.**

| **Polysaccharide Hydrogel** | **SH-HP-HA** | **SH-AB-HA** | **SH-HE-HA** | **SH-CH-HA** | **Control SH-HA-1** |
|---|---|---|---|---|---|
| Breaking strain (%) | 80.5% | 74.2% | 70.8% | 72.5% | 59.3% |
| Breaking stress (kPa) | 520 | 550 | 610 | 580 | 600 |

| **Polysaccharide Hydrogel** | **SH-SU-CTS** | **SH-HP-CTS** | **SH-CM-CT** | **SH-HE-CTS** | **Control SH-HA-2** |
|---|---|---|---|---|---|
| Breaking strain (%) | 82.6% | 75.1% | 72.5% | 70.5% | 56.2% |
| Breaking stress (kPa) | 560 | 670 | 700 | 710 | 680 |

The thiolated hyaluronic acid derivative hydrogels and thiolated chitin/chitosan derivative hydrogels prepared in Examples 1-8 have good transparency. The compressibility of these hydrogels is significantly higher than that of the thiolated hyaluronic acid hydrogel prepared without grafting side chain groups. This suggests that the thiolated hyaluronic acid derivative hydrogels and thiolated chitin/chitosan derivative hydrogels have better flexibility, self-healing capability, and resilience. The flexible hydrogels, as a substitute for the vitreous body, can enhance shock absorption function. The excellent self-healing capability and resilience can maintain the shape of the eyeball and keep the pressure on the retina to maintain it in the correct position.

### Example 11: Measurement of degradation performance of the hydrogels

Hydrogels prepared in examples 3, 5-8, and comparative example 2 were made into cylindrical shapes with a diameter of 8 mm and a height of 10 mm. 4 gel blocks were prepared from each of the 6 hydrogels. Two of the gel blocks are dried using a freeze dryer, and their weights are measured and averaged to obtain the initial weight before enzymatic degradation. The other 2 gel blocks were immersed in 3 ml of PBS solution (pH 7.4) containing 10000 U/ml of lysozyme. The enzyme solution was changed every 3 days, and this process was continued for 30 days. At the end of the 30-day period, the enzyme solution was filtered together with the remaining gel blocks using pre-weighed filter paper. The filter paper and gel blocks were washed with PBS solution three times and then dried using a freeze dryer. The weights of the dried filter paper and gel blocks were measured, and the weight of the filter paper was subtracted to obtain the average weight after enzymatic degradation. The degradation rate was calculated based on these measurements and recorded in Table 2. From Table 2, it can be observed that under the experimental conditions, the degradation rate of thiolated hyaluronic acid hydrogel is 73.3%, while the degradation rates of thiolated polysaccharide derivatives hydrogels range from 46% to 66%. These degradation rates are significantly lower than that of thiolated hyaluronic acid, indicating that thiolated polysaccharide derivatives hydrogels have better resistance to degradation.

### In vitro degradation of thiolated polysaccharide hydrogels:

| **Polysaccharide Hydrogel** | **SH-AB-HA** | **SH-SU-CTS** | **SH-HP-CTS** | **SH-CM-CT** | **SH-HE-CTS** | **Comparative SH-HA-2** |
|---|---|---|---|---|---|---|
| Degradation Rate | 66.2% | 45.8% | 52.3% | 56.4% | 65.5% | 73.3% |

### Example 12: Use of thiolated hyaluronic acid derivative hydrogels as intraocular vitreous substitutes

Six female New Zealand rabbits weighing 2.5-3kg were used as experimental animals. They were divided into SH-HP-HA hydrogel group and SH-AB-HA hydrogel group, with three rabbits in each group. The left eye of each rabbit was the operated eye, while the right eye served as the control eye.

Following the methods described in Examples 1 and 3, thiolated hydroxypropyl hyaluronic acid and thiolated aminobutyric hyaluronic acid, which had been sterilized by irradiation, were prepared respectively into SH-HP-HA gel solution with a concentration of 2.5% and SH-AB-HA gel solution with a concentration of 4.5%, under aseptic conditions using sterile containers and liquids.

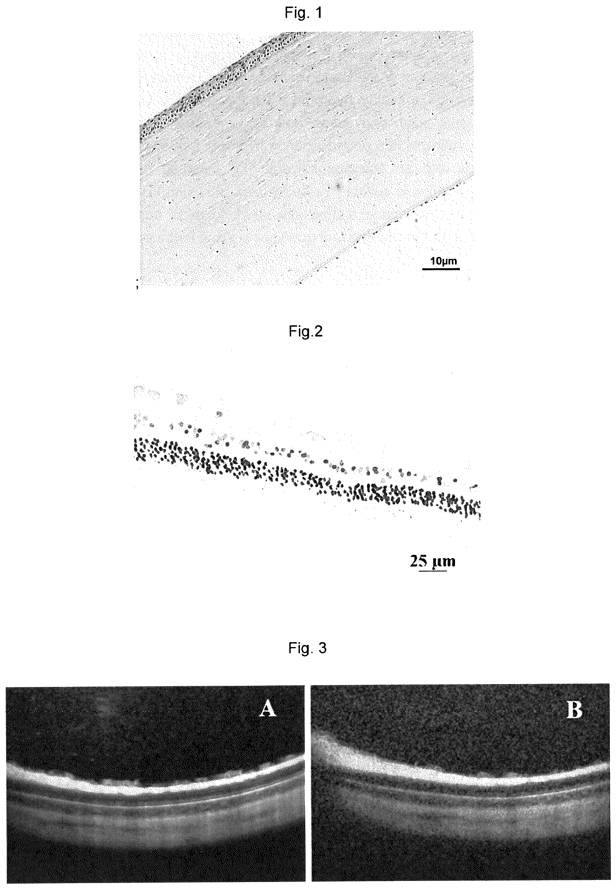

The experimental animals were anesthetized and the skin was disinfected according to routine procedures. Vitrectomy was performed, and the SH-HP-HA gel solution was injected into the vitreous cavity of the left eyes of the SH-HP-HA hydrogel group, while the SH-AB-HA gel solution was injected into the vitreous cavity of the left eyes of the SH-AB-HA hydrogel group. The gel formed in situ and the conjunctival incision was closed. The postoperative care of the rabbits was conducted, and they were kept under normal conditions. Slit lamp examination and B-mode ultrasound examination were conducted 90 days after the surgery. The eyeballs were collected for histopathological examination with HE staining (as shown in Figures 1 and 2) to evaluate the filling of the hydrogel in the vitreous cavity.

Postoperative slit lamp examination showed mild inflammation reaction in the operated eyes of the experimental rabbits, with clear visibility of the fundus. On day 90 after surgery, the cornea of the operated eyes showed no swelling, the vitreous remained transparent, and the retinal vessels were clear. B-mode ultrasound examination revealed no foreign bodies, opacities, hemorrhages, or retinal detachments in the vitreous, and there were no significant differences compared to normal eyes. On day 90 after surgery, the operated eyes and normal eyes were dissected, embedded in paraffin, sectioned, and stained with HE for observation. Comparing the operated eyes with the normal eyes, the cornea of the operated eyes showed no significant pathological changes and had a normal and intact structure. The retinal layers were intact, and the arrangement of the cone and rod cells was compact. The experimental results showed that the prepared thiolated hyaluronic acid derivative hydrogel had no irritation in the eyes and exhibited good compatibility with ocular tissues. It demonstrated excellent vitreous substitute effects, with the ability to support the retina and maintain the shape of the eyeball, and had good optical properties. It could degrade and absorb within the eye. Thiolated hyaluronic acid polysaccharide derivative hydrogel has great potential as an intraocular vitreous substitute.

### Example 13: Use of thiolated chitin/chitosan derivative hydrogels as intraocular vitreous substitutes

Three male New Zealand rabbits weighing 2.8-3kg were used as experimental animals. The left eye of each rabbit was the operated eye, while the right eye served as the control eye. The vitreous substitute effect of the thiolated hydroxypropyl chitosan hydrogel prepared in Example 6 was assessed.

Following the method described in Example 6, a thiolated hydroxypropyl chitosan gel with a concentration of 4% was aseptically prepared using sterilized thiolated hydroxypropyl chitosan. New Zealand rabbits were intravenously anesthetized and the skin was disinfected. Vitrectomy surgery was performed on the left eye of each rabbit, and the 4% thiolated hydroxypropyl chitosan gel was injected into the vitreous cavity to form a gel in situ. The conjunctival incision was closed, and postoperative care was provided to the rabbits, which were then kept under normal conditions. On day 90 after the surgery, slit lamp examination, B-scan ultrasound, and optical coherence tomography (OCT) were performed to evaluate the filling of the hydrogel in the vitreous cavity.

On day 90 after surgery, the operated eyes of the rabbits showed mild inflammatory reactions, with a transparent vitreous and clear visibility of the fundus and retinal vessels during slit lamp examination. B-scan ultrasound did not detect any foreign bodies, opacities, hemorrhages, or retinal detachments, and there were no significant differences compared to normal eyes. As shown in Figure 3, OCT examination revealed no retinal or choroidal detachments, no edema, and transparent refractive media, with no significant differences compared to normal eyes. The experimental results demonstrated that the prepared thiolated chitin/chitosan derivative hydrogel had no eye irritation, exhibited good compatibility with ocular tissues, and had excellent vitreous substitution effects. It could exert pressure on the retina and maintain the shape of the eyeball, with good optical properties. The hydrogel could degrade and absorb within the eye. Therefore, the thiolated chitin/chitosan derivative hydrogel has great application potential as an intraocular vitreous substitute.

Unless otherwise specified, the agents and materials used in this article are commercially available or can be prepared using known methods. For example:
Hydroxypropyl hyaluronic acid, hydroxyethyl hyaluronic acid, aminobutyric hyaluronic acid, and hydrazide hyaluronic acid are commercially available from Qingdao Boyite Biotechnology Co., Ltd.

Succinyl chitosan, hydroxypropyl chitosan, carboxymethyl chitosan, and hydroxyethyl chitosan are commercially available from Qingdao Honghai Biotechnology Co., Ltd.

Hyaluronic acid is commercially available from Huaxi Biotechnology Co., Ltd.

The testing instruments and conditions used in the examples are as follows:

### (1) Transmittance measurement

Instrument model: UV-Vis spectrophotometer TU-1810, Beijing Puxi General Instrument Co., Ltd.

Testing conditions: Measure the absorbance within the wavelength range of 400-800 nm at room temperature, and calculate transmittance using the formula: transmittance (%) = 10 ^{2-absorbance} × 100%. Measure 5 parallel samples and take the average value.

### (2) Compression performance measurement

Instrument model: Universal electronic tensile testing machine AGS-X, Shimadzu Corporation, Japan
Testing conditions: The hydrogel is prepared into cylindrical shape with a diameter of 0.8 cm and a height of 0.8 cm. Perform compression performance test at room temperature with a compression rate of 3 mm/min. Measure 5 parallel samples and take the average value.

### (3) HE staining observation of tissue sections

Instrument model: Slicer RM2016, Shanghai Leica Instrument Co., Ltd.

Testing conditions: Fix the obtained tissues with 4% neutral formaldehyde, dehydrate them with ethanol gradient, embed them in conventional tissues, and then perform paraffin sections with a thickness of 4 µ m. Observe the sections under a microscope after staining with hematoxylin eosin (H&E).

### (4) B-mode ultrasonography

Instrument model: Ophthalmic B-mode ultrasound instrument SW-2100, Tianjin Soweie
Testing conditions: Apply coupling agent to the outer skin of the rabbit eyelids at room temperature and use a B-mode ultrasound probe to obtain ultrasound images of the eye.

### (5) Optical coherence tomography (OCT) examination

Instrument model: Optical coherence tomography scanner Optovue Avanti, Visionix, USA
Testing conditions: Perform surface anesthesia on the rabbit eyes using proparacaine eye drops, place an eyelid opener inside the eyelids to fully expose the eyeball, then perform retinal layer scanning to the rabbit eyes using the optical coherence tomography scanner.

## Claims

1. A thiolated polysaccharide derivative hydrogel for use as an intraocular vitreous substitute, selected from:
(i) a hydrogel formed from a thiolated hyaluronic acid derivative and an aqueous solvent, wherein the mass percentage concentration of the thiolated hyaluronic acid derivative in the aqueous solvent is 3% to 6%, and the thiol substitution degree of the thiolated hyaluronic acid derivative is 3% to 20%, and wherein the hyaluronic acid derivative is selected from the group consisting of hydroxyethyl hyaluronic acid, hydroxypropyl hyaluronic acid, acetyl hyaluronic acid, butyryl hyaluronic acid, aminoacetic hyaluronic acid, aminopropionic hyaluronic acid, aminobutyric hyaluronic acid, and hydrazide hyaluronic acid; and
(ii) a hydrogel formed from a thiolated chitin/chitosan derivative and an aqueous solvent, wherein the mass percentage concentration of the thiolated chitin/chitosan derivative in the aqueous solvent is 2% to 10%, and the thiol substitution degree of the thiolated chitin/chitosan derivative is 3% to 25%, and wherein the chitin/chitosan derivative is selected from the group consisting of carboxyethyl chitosan, hydroxyethyl chitosan, hydroxypropyl chitosan, hydroxybutyl chitosan, succinyl chitosan, aminoacetic chitosan, aminopropionic chitosan, aminobutyric chitosan, hydroxyethyl succinyl chitosan, hydroxypropyl succinyl chitosan, hydroxybutyl succinyl chitosan, carboxymethyl chitin, and carboxyethyl chitin.

2. The hydrogel for use according to claim 1, wherein the aqueous solvent is selected from the group consisting of water, physiological saline, phosphate buffer, physiological balanced solution and glucose solution.

3. An intraocular vitreous substitute comprising a hydrogel according to claim 1or 2.

4. A thiolated polysaccharide derivative for use as an intraocular vitreous substitute, selected from:
(i) a thiolated hyaluronic acid derivative having a thiol substitution degree of 3% to 20%, wherein the hyaluronic acid derivative is selected from the group consisting of hydroxyethyl hyaluronic acid, hydroxypropyl hyaluronic acid, acetyl hyaluronic acid, butyryl hyaluronic acid, aminoacetic hyaluronic acid, aminopropionic hyaluronic acid, aminobutyric hyaluronic acid, and hydrazide hyaluronic acid; and
(ii) a thiolated chitin/chitosan derivative having a thiol substitution degree of 3% to 25%, wherein the chitin/chitosan derivative is selected from the group consisting of carboxyethyl chitosan, hydroxyethyl chitosan, hydroxypropyl chitosan, hydroxybutyl chitosan, succinyl chitosan, aminoacetic chitosan, aminopropionic chitosan, aminobutyric chitosan, hydroxyethyl succinyl chitosan, hydroxypropyl succinyl chitosan, hydroxybutyl succinyl chitosan, carboxymethyl chitin, and carboxyethyl chitin.

5. A method for preparing a hydrogel for an intraocular vitreous substitute, comprising the following steps: dissolving the thiolated polysaccharide derivative according to claim 4 in an aqueous solvent to prepare a gel solution, wherein the gel solution is subjected to self-crosslinking to obtain the hydrogel, and the aqueous solvent is selected from the group consisting of water, physiological saline, phosphate buffer, physiological balanced solution and glucose solution, and wherein
the thiolated polysaccharide derivative is a thiolated hyaluronic acid derivative, and wherein the thiolated hyaluronic acid derivative is dissolved in an aqueous solvent to prepare a gel solution with a concentration of 3-6%, adjusting the pH to 7.2-8.5, and the gel solution is subjected to self-crosslinking to obtain a hydrogel; or
the thiolated polysaccharide derivative is a thiolated chitin/chitosan derivative, and wherein the thiolated chitin/chitosan derivative is dissolved in an aqueous solvent to prepare a gel solution with a concentration of 2-10%, adjusting the pH to 7.0-8.5, and the gel solution is subjected to self-crosslinking to obtain a hydrogel.

## Patentansprüche

1. Thioliertes Polysaccharidderivat-Hydrogel zur Verwendung als intraokularer Glaskörperersatz, ausgewählt aus:
(i) einem Hydrogel, gebildet aus einem thiolierten Hyaluronsäurederivat und einem wässrigen Lösungsmittel, wobei die Massenprozentkonzentration des thiolierten Hyaluronsäurederivats in dem wässrigen Lösungsmittel 3 % bis 6 % beträgt und der Thiol-Substitutionsgrad des thiolierten Hyaluronsäurederivats 3 % bis 20 % beträgt, und wobei das Hyaluronsäurederivat ausgewählt ist aus der Gruppe bestehend aus Hydroxyethylhyaluronsäure, Hydroxypropylhyaluronsäure, Acetylhyaluronsäure, Butyrylhyaluronsäure, Aminoessigsäure-Hyaluronsäure, Aminopropionyl-Hyaluronsäure, Aminobuttersäure-Hyaluronsäure und Hydrazid-Hyaluronsäure; und
(ii) einem Hydrogel, gebildet aus einem thiolierten Chitin-/Chitosan-Derivat und einem wässrigen Lösungsmittel, wobei die Massenprozentkonzentration des thiolierten Chitin-/Chitosan-Derivats in dem wässrigen Lösungsmittel 2 % bis 10 % beträgt und der Thiol-Substitutionsgrad des thiolierten Chitin-/ChitosanDerivats 3 % bis 25 % beträgt, und wobei das Chitin-/Chitosan-Derivat ausgewählt ist aus der Gruppe bestehend aus Carboxyethylchitosan, Hydroxyethylchitosan, Hydroxypropylchitosan, Hydroxybutylchitosan, Succinylchitosan, Aminoessigsäure-Chitosan, Aminopropionyl-Chitosan, Aminobuttersäure-Chitosan, Hydroxyethylsuccinylchitosan, Hydroxypropylsuccinylchitosan, Hydroxybutylsuccinylchitosan, Carboxymethylchitin und Carboxyethylchitin.

2. Hydrogel zur Verwendung nach Anspruch 1, wobei das wässrige Lösungsmittel ausgewählt ist aus der Gruppe bestehend aus Wasser, physiologischer Kochsalzlösung, Phosphatpuffer, physiologischer balancierter Lösung und Glucoselösung.

3. Intraokularer Glaskörperersatz, umfassend ein Hydrogel nach Anspruch 1 oder 2.

4. Thioliertes Polysaccharidderivat zur Verwendung als intraokularer Glaskörperersatz, ausgewählt aus:
(i) einem thiolierten Hyaluronsäurederivat mit einem Thiol-Substitutionsgrad von 3 % bis 20 %, wobei das Hyaluronsäurederivat ausgewählt ist aus der Gruppe bestehend aus Hydroxyethylhyaluronsäure, Hydroxypropylhyaluronsäure, Acetylhyaluronsäure, Butyrylhyaluronsäure, Aminoessigsäure-Hyaluronsäure, Aminopropionyl-Hyaluronsäure, Aminobuttersäure-Hyaluronsäure und Hydrazid-Hyaluronsäure; und
(ii) einem thiolierten Chitin-/Chitosan-Derivat mit einem Thiol-Substitutionsgrad von 3 % bis 25 %, wobei das Chitin-/Chitosan-Derivat ausgewählt ist aus der Gruppe bestehend aus Carboxyethylchitosan, Hydroxyethylchitosan, Hydroxypropylchitosan, Hydroxybutylchitosan, Succinylchitosan, Aminoessigsäure-Chitosan, Aminopropionyl-Chitosan, Aminobuttersäure-Chitosan, Hydroxyethylsuccinylchitosan, Hydroxypropylsuccinylchitosan, Hydroxybutylsuccinylchitosan, Carboxymethylchitin und Carboxyethylchitin.

5. Verfahren zur Herstellung eines Hydrogels für einen intraokularen Glaskörperersatz, umfassend die folgenden Schritte: Lösen des thiolierten Polysaccharidderivats nach Anspruch 4 in einem wässrigen Lösungsmittel zur Herstellung einer Gellösung, wobei die Gellösung einer Selbstvernetzung unterzogen wird, um das Hydrogel zu erhalten, und das wässrige Lösungsmittel ausgewählt ist aus der Gruppe bestehend aus Wasser, physiologischer Kochsalzlösung, Phosphatpuffer, physiologischer balancierter Lösung und Glucoselösung, und wobei
das thiolierte Polysaccharidderivat ein thioliertes Hyaluronsäurederivat ist und wobei das thiolierte Hyaluronsäurederivat in einem wässrigen Lösungsmittel gelöst wird, um eine Gellösung mit einer Konzentration von 3-6 % herzustellen, der pH-Wert auf 7,2-8,5 eingestellt wird und die Gellösung einer Selbstvernetzung unterzogen wird, um ein Hydrogel zu erhalten; oder
das thiolierte Polysaccharidderivat ein thioliertes Chitin-/Chitosan-Derivat ist und wobei das thiolierte Chitin-/Chitosan-Derivat in einem wässrigen Lösungsmittel gelöst wird, um eine Gellösung mit einer Konzentration von 2-10 % herzustellen, der pH-Wert auf 7,0-8,5 eingestellt wird und die Gellösung einer Selbstvernetzung unterzogen wird, um ein Hydrogel zu erhalten.

## Revendications

1. Hydrogel à base de dérivé de polysaccharide thiolé pour son utilisation en tant que substitut du vitré intra-oculaire, choisi parmi :
(i) un hydrogel formé à partir d'un dérivé d'acide hyaluronique thiolé et d'un solvant aqueux, dans lequel la concentration, en pourcentage en masse, du dérivé d'acide hyaluronique thiolé dans le solvant aqueux est de 3 % à 6 %, et le degré de substitution thiol du dérivé d'acide hyaluronique thiolé est de 3 % à 20 %, et dans lequel le dérivé d'acide hyaluronique est choisi dans le groupe constitué par l'acide hyaluronique hydroxyéthylé, l'acide hyaluronique hydroxypropylé, l'acide hyaluronique acétylé, l'acide hyaluronique butyrylé, l'acide hyaluronique aminoacétique, l'acide hyaluronique aminopropionique, l'acide hyaluronique aminobutyrique, et l'acide hyaluronique hydrazide ; et
(ii) un hydrogel formé à partir d'un dérivé de chitosane/chitine thiolé et d'un solvant aqueux, dans lequel la concentration, en pourcentage en masse, du dérivé de chitosane/chitine thiolé dans le solvant aqueux est de 2 % à 10 %, et le degré de substitution thiol du dérivé de chitosane/chitine thiolé est de 3 % à 25 %, et dans lequel le dérivé de chitosane/chitine est choisi dans le groupe constitué par le chitosane carboxyéthylé, le chitosane hydroxyéthylé, le chitosane hydroxypropylé, le chitosane hydroxybutylé, le chitosane succinylé, le chitosane aminoacétique, le chitosane aminopropionique, le chitosane aminobutyrique, le chitosane hydroxyéthyl-succinylé, le chitosane hydroxypropyl-succinylé, le chitosane hydroxybutyl-succinylé, la chitine carboxyméthylée, et la chitine carboxyéthylée.

2. Hydrogel pour son utilisation selon la revendication 1, dans lequel le solvant aqueux est choisi dans le groupe constitué par l'eau, une solution saline physiologique, un tampon phosphate, une solution équilibrée physiologique et une solution de glucose.

3. Substitut du vitré intra-oculaire comprenant un hydrogel de la revendication 1 ou 2.

4. Dérivé de polysaccharide thiolé pour son utilisation en tant que substitut du vitré intra-oculaire, choisi parmi :
(i) un dérivé d'acide hyaluronique thiolé ayant un degré de substitution thiol de 3 % à 20 %, et dans lequel le dérivé d'acide hyaluronique est choisi dans le groupe constitué par l'acide hyaluronique hydroxyéthylé, l'acide hyaluronique hydroxypropylé, l'acide hyaluronique acétylé, l'acide hyaluronique butyrylé, l'acide hyaluronique aminoacétique, l'acide hyaluronique aminopropionique, l'acide hyaluronique aminobutyrique, et l'acide hyaluronique hydrazide ; et
(ii) un dérivé de chitosane/chitine thiolé ayant un degré de substitution thiol de 3 % à 25 %, et dans lequel le dérivé de chitosane/chitine est choisi dans le groupe constitué par le chitosane carboxyéthylé, le chitosane hydroxyéthylé, le chitosane hydroxypropylé, le chitosane hydroxybutylé, le chitosane succinylé, le chitosane aminoacétique, le chitosane aminopropionique, le chitosane aminobutyrique, le chitosane hydroxyéthyl-succinylé, le chitosane hydroxypropyl-succinylé, le chitosane hydroxybutyl-succinylé, la chitine carboxyméthylée, et la chitine carboxyéthylée.

5. Méthode de préparation d'un hydrogel pour un substitut du vitré intra-oculaire, comprenant les étapes suivantes : dissolution du dérivé de polysaccharide thiolé selon la revendication 4 dans un solvant aqueux afin de préparer une solution de gel, dans laquelle la solution de gel est soumise à une auto-réticulation afin d'obtenir l'hydrogel, et le solvant aqueux est choisi dans le groupe constitué par l'eau, une solution saline physiologique, un tampon phosphate, une solution équilibrée physiologique et une solution de glucose, et dans laquelle
le dérivé de polysaccharide thiolé est un dérivé d'acide hyaluronique thiolé, et dans laquelle le dérivé d'acide hyaluronique thiolé est dissous dans un solvant aqueux afin de préparer une solution de gel avec une concentration de 3 à 6 %, le pH est ajusté à 7,2-8,5, et la solution de gel est soumise à une auto-réticulation afin d'obtenir un hydrogel ; ou
le dérivé de polysaccharide thiolé est un dérivé de chitosane/chitine thiolé, et dans lequel le dérivé de chitosane/chitine thiolé est dissous dans un solvant aqueux afin de préparer une solution de gel avec une concentration de 2 à 10%, le pH est ajusté à 7,0-8,5, et la solution de gel est soumise à une auto-réticulation afin d'obtenir un hydrogel.
